# EUROPEAN PATENT APPLICATION

(11) **EP 3 023 420 A1**
(43) Date of publication of application: **25.05.2016**
(21) Application number: 14193655.9
(22) Date of filing: 18.11.2014
(51) Int. Cl.: C07D 403/14, C07D 209/44, G01N 33/543, G01N 33/58, G01N 21/64, C12Q 1/68

(54) **Methods for providing stable isoindole derivatives**

(71) Applicant: Fraunhofer-Gesellschaft zur Förderung der angewandten Forschung e.V., 80686 München (DE)
(72) Inventor: Schramm, Oana Genoveva, 50259 Pulheim (DE); Di Fiore, Stefano, 41460 Neuss (DE); Fischer, Rainer, 52076 Aachen (DE)
(74) Representative: Roth, Andy Stefan

(57) **Abstract**

The technology provided herein relates to a method for producing an isoindole derivative, the isoindole derivative obtainable by this method, a method for stabilizing an isoindole derivative and a method for detection and/or quantification of macromolecules.

## Description

### FIELD OF THE DISCLOSURE

The technology provided herein relates to a method for producing an isoindole derivative, the isoindole derivative obtainable by this method, a method for stabilizing an isoindole derivative and a method for detection and/or quantification of macromolecules.

### BACKGROUND

Detection and functionalization of synthetic or naturally occurring macromolecules are essential tools for rational design and synthesis of new safe and efficient nanomaterials. Since most of these macromolecules lack intrinsic detectable properties it is difficult to monitor their pharmacokinetics. To date, the functionalization of macromolecules with chromophores and/or fluorophores suffers from several drawbacks, i.e. low degree of functionalization, relatively low solubility of the products, long reaction times, and harsh derivatization conditions (Q. Zhang, Y. Sha, J.-H. Wang Molecules, 2010, 15, 2969-2971 and B.-B. Wang, X. Zhang, X.-R. Jia, Z.-C. Li, Y. Ji, L. Yang, Y. Wie J. Am. Chem. Soc. 2004, 126, 15180-15194).

Detection of small molecules bearing primary amino groups such as aminoacids and biogenic amines can be readily achieved by their reaction with o-phthaldialdehyde (OPA) and thiols with the formation of isoindole derivatives (M. Roth, Anal. Chem. 1971, 43, 880-882) (Scheme 1).

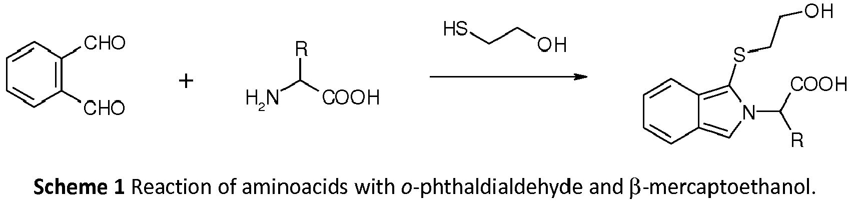

Despite of its simplicity, almost quantitative conversion, mild reaction conditions, the method has been almost exclusively used for detection purposes since the resulting isoindoles are instable, with half-life times below 1 h (P. Zuman, Chem. Rev. 2004, 104, 3217-3238, D. P. Manica, J. A. Lapos, A. D. Jones, A. G. Ewing Anal. Biochem. 2003, 322, 68-78, W.A. Jacobs, M.W. Leburg, E.J. Madaj, Anal. Biochem. 1986, 156, 334-340).

The main application area of this reaction is the detection of amino acids using OPA and different thiols as a pre-column derivatization method for reversed phase chromatography (R. Hanczkó, A. Jámbor, A. Perl, I. Molnár-Perl J. Chromatogr. A 2007, 1163, 25-42). More recently this method has been extended to other biogenic amines, small peptides and some proteins, where the thiol component contains an ionizable moiety that facilitates the detection by mass spectrometry. However the stability of these isoindoles was not addressed (T. I. F. Cremers, J. L. A. Anna De, W. S. Faber, Method for the determination of an analyte comprising a primary amino group, and kit for labeling said analyte, WO2008094043 A2, 2008, PCT/NL2008/050155, O. Richard, R. Gavin, S. Richard, Method for analysing amino acids, peptides and proteins using mass spectroscopy of fixed charge-modified derivatives, WO 2004046731 A3, 2005, PCT/US2003/036739).

Several attempts to stabilize the isoindole heterocycles have been carried out by using moderate excess of fluorogenic and/or thiol components or highly substituted thiols or by replacing the thiol nucleophilic component for a cyanide. However, increasing the excess of OPA and/or thiol component above moderate levels had only limited effect on the stability of the isoindoles. In general a rather negative effect of high excess of these components on the stability of the isondole has been reported. The replacement of thiols with cyanide did not increase the stability of the corresponding isoindoles, their half-lifes of ca. 2 min. being comparable with that of β-mercaptoethanol products (D. P. Manica, J. A. Lapos, A. D. Jones, A. G. Ewing Anal. Biochem. 2003, 322, 68-78).

Therefore the availability of methods that can stabilize the isoindole derivatives would be highly advantageous for functionalization and for detection purposes.

### SUMMARY OF THE DISCLOSURE

The present disclosure pertains to methods for derivatization of macromolecules containing primary amino groups in an one-pot fashion using a three component reaction system with the formation of isoindole derivatives, wherein the drawbacks of the prior art can be avoided.

In a first aspect, embodiments of the disclosure relate to a method for producing an isoindole derivative, wherein a Component I having the formula 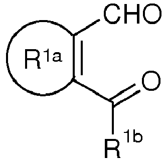 in which
R^{1b} is H, C1 to C4 alkyl group or an electron deficient aromatic group, and 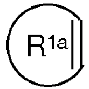
is an electron deficient π-system, an electron neutral π-system or an electron rich π-system,
is reacted in an one-pot-reaction with, as Component II, a macromolecule having at least one primary amino group or an amino functionalized surface and, as Component III a thiol reagent or a thiol functionalized surface.

In a further aspect, embodiments of the present disclosure relate to an isoindole derivative obtainable by the above described method.

In another aspect, embodiments of the present disclosure relate to a method for stabilizing an isoindole derivative by producing an isoindole derivative according to the method as mentioned above.

In a yet further aspect, embodiments of the present disclosure relate to a method for detecting and/or quantifying a macromolecule comprising a primary amino group, wherein the method comprises:
a) obtaining a stabilized isoindole derivative containing the macromolecule to be detected and/or analyzed according to the method as defined above; and
b) detecting and/or quantifying the isoindole derivative.

Before the disclosure is described in detail, it is to be understood that the terminology used herein is for purposes of describing particular embodiments only, and it is not intended to be limiting. It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

### BRIEF DESCRIPTION OF THE DRAWINGS

Figure 1 is a diagram showing the formation kinetic and stability of different isoindoles reaction products of various amino containing molecules with OPA and NAC.
Figure 2 is a diagram showing the formation kinetic and stability of different isoindoles reaction products of various amino containing molecules with OPA and PEGSH₈₀₀.
Figure 3 is a diagram showing the formation kinetic and stability of different isoindoles reaction products of various amino containing molecules with MOPAC and NAC.
Figure 4 is a diagram showing the formation kinetic and stability of different isoindoles reaction products of butylamine and various components I and III.
Figure 5 is a diagram showing the SE-FPLC chromatograms of PAMAM dendrimers of different generations (G0-G5) derivatized with PEG-SH₈₀₀ and OPA.
Figure 6 is a diagram showing the SE-FPLC chromatograms of different concentrations of PAMAM G3 derivatized with PEG-SH₈₀₀ and OPA.
Figure 7 is a diagram showing the quantification of PAMAM G3 by SEC-HPLC using the three components reaction of the present disclosure.

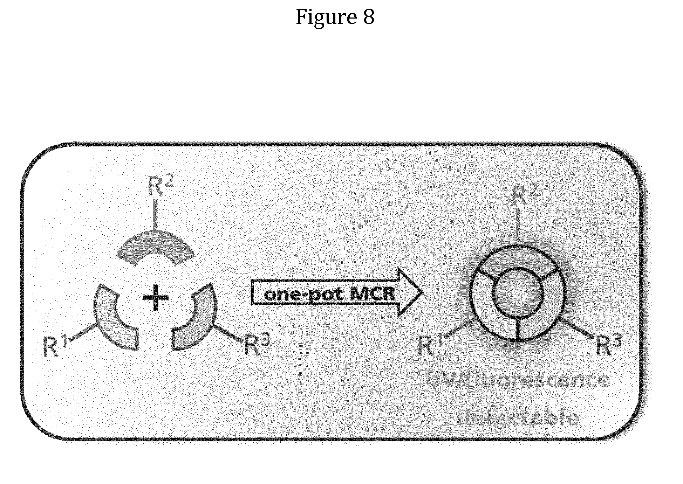

Figure 8 is a scheme of the one-pot reaction using a three component reaction system according to the present disclosure.

### DETAILED DESCRIPTION OF THIS DISCLOSURE

The present disclosure pertains to methods for derivatization of macromolecules containing primary amino groups in an one-pot reaction using a three component reaction with the formation of isoindole derivatives.

The reaction between primary amines, o-phthaldialdehyde (OPA) and thiols has already been used in analytical chemistry for the detection of aminoacids (see WO 2004/046731 A2 and WO 2008/094043 A2). However, due to the intrinsic instability of the resulting isoindoles the synthetic potential of this reaction has not been fully exploited so far.

In one aspect, a method is disclosed for producing an isoindole derivative, wherein a Component I having the formula 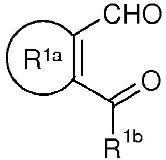 in which
R^{1b} is H, C1 to C4 alkyl group or an electron deficient aromatic group, such as phenyl, and 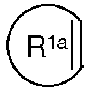
is an electron deficient π-system, an electron neutral π-system or an electron rich π-system,
is reacted in an one-pot-reaction with, as Component II, a macromolecule having at least one primary amino group or an amino functionalized surface and, as Component III a thiol reagent.

As mentioned above, the group R^{1a} is a π-system. As used herein, the term "π-system" means that a n-bond is present. This can be a single or conjugated n-bond. The n-bond can be present for example in an aromatic compound.

The term "electron deficient" as used herein refers to the property of withdrawing electrons. This means in turn that the term "electron rich" means the property of providing electrons and the term "electron neutral" refers to substituents, which are neither electron deficient nor electron rich.

Preferably the electron deficient π-system is selected from the group consisting of pyridine, pyrimidine, benzoic acid, benzonitrile, (1,1',1"-trifluoromethyl)benzene, benzeneboronic acid, nitrobenzene, chlorobenzene, quinoline, isoquinoline, naphthyridine. The electron neutral π-system is preferably benzene or naphthalene. The electron rich π-system is preferably selected from the group consisting of toluene, thiophene, phenol, furan, pyrrole, N,N-dimethylaniline. In particular, Component I is selected from the group consisting of 2,3-pyridinedicarboxyaldehyde, 3,4-diformyl-benzoic acid, 2-acetylthiophene-3-carboxyaldehyd or 4-(2-formylquinoline-3-carbonyl)benzoic acid.

In the above described method, Component II is a macromolecule having at least one amino group. A macromolecule is a molecule commonly created by polymerization of smaller subunits, preferably at least three subunits. The individual constituent molecules of polymeric macromolecules are called monomers. A macromolecule can be as well a non-polymeric macrocycle (defined as any molecule containing a ring of twelve or more atoms). Typical examples of biomacromolecules are peptides, proteins, DNA, RNA, carbohydrates. In the method as defined above, the macromolecule can be any kind of macromolecule having at least one primary amino group, preferably at least four amino groups.

Preferred examples of the macromolecule as component II are selected from the group consisting of polylysine, poly(amidoamine) dedrimers (PAMAM), polypropylene imine dendrimers (PPI), poly(melamine) dendrimers, peptides, proteins, such as albumin, and sugars.

An amino functionalized surfaces can be used as Component II in the method for producing an isoindole derivative. The term "amino functionalized surface" as used herein refers to the surface of any solid body having at least one primary amino group. Said solid bodies can be made of any solid material, in particular glass or polymers, especially in the form of membranes. The at least one primary amino group can be attached to the surface by any means known to the skilled person, preferably by a covalent bond. Such amino fuctionalized surfaces are known to the skilled person in the form of membranes or as glasses to be used in the chip technology.

As mentioned above, as Component III a thiol is used. In the disclosed method, any thiol can be used that has no functional groups, such as primary amino groups, interfering with the Component I and II during the isoindole derivative formation.

Such a thiol can be a bulky thiol having a molecular weight of in particular 200 Da or more or any thiol with a molecular weight of less than in particular 200 Da having any substituent in position α and/or β and/or γ to the thiol group, e.g. β-mercaptoethanol.

Preferably the thiol as Component III contains at least one thiol group and no primary amino group.

A thiol functionalized surfaces can be used as Component III in the methods for producing an isoindole derivative according to the present disclosure. The term "thiol functionalized surface" as used herein refers to the surface of any solid body having at least one thiol group. Said solid bodies can be made of any solid material, in particular gold, glass, polymers, or membranes. The at least one thiol group can be attached to the surface by any means known to the skilled person, preferably by a covalent bond. Such amino fuctionalized surfaces are known to the skilled person in the form of membranes, gold nanoparticles or as glasses to be used in the chip technology.

The method as described above is carried out as one-pot-reaction. The term "one-pot-reaction" as used herein means a chemical reaction, wherein all three Components I, II, and III as well as solvents, if necessary, are mixed together in one reaction vessel and the reaction is started after components I, II, and III are mixed together.

The method for producing the isoindole derivative as described above can be carried out in a solvent. A solvent as used herein means any liquid, which can at least partially solve Components I, II, and III as described above. Such a solvent can have a basic pH, i.e. a pH value greater than 7, in particular a pH value of 8 and higher. If necessary, the pH value of the solvent can be adjusted to the required pH by adding an acid or an alkali. Alternatively, a buffer can be used having the desired pH value. Examples of the solvent are water, ethanol, dimethylsulfoxide, biological fluids, like serum, blood, saliva, urine, milk, juices, and mixtures of two or more solvents.

Preferably, the reaction comprises the steps of:
a) adding a solution of Component III to component II that can be either in solution or in solid phase (amino functionalized surface), or in case that component III is in solid phase (thiol functionalized surface) a solution of component II is added to component III,
b) adding Component I to said mixture obtained in step a), and
c) incubating the mixture of Component I, II and III obtained after step b) in order to obtain the isoindole derivative. That is, the reaction is started by the incubation when the three Components I, II, and II are present in the reaction vessel.

In a preferred embodiment the Components are mixed in a ratio of 1 : 1.1 : 1.5 with respect to the equivalents of amino groups of Component II : equivalents of Component I : equivalents of Component III.

In another preferred embodiment, the Components are mixed with a larger excess of Component I and III, preferably in a ration 1 : >5 : >7.5 with respect to the equivalents of amino groups of Component II : equivalents of Component I : equivalents of Component III, respectively.

Preferably, the reaction of Component I, II and III can be carried out in an aqueous solution having a pH value greater than 7 and preferably having a pH of 8 or higher. The pH is adjusted according to the method explained above.

The method can be carried out at a temperature in a range from 0 °C to 80 °C, preferably room temperature.

According to the above described method, in particular the following compounds:
a) Component I is 3,4-diformyl-benzoic acid methyl ester (MOPAC), or o-phthaldialdehyde (OPA),
b) Component II is a protein or a dendrimer like a poly(amidoamine) dendrimer
c) Component III is β-mercaptoethanol (βMEtOH), N-acetyl-L-cysteine (NAC), poly(ethylene glycol) methyl ether thiol having a molecular weight of about 800 or higher.

Particular suitable combinations of Components in the method for producing an isoindole derivative are the following
a) Component I wherein R^{1a} is an electron deficient group, Component II is a macromolecule having at least four amino groups and Component III is a bulky thiol.
b) Component I wherein R^{1a} is an electron deficient, an electron donating or an electron neutral group, Component II is a macromolecule with at least four amino groups and Component III is a bulky thiol.
c) Component I wherein R^{1a} is an electron deficient group, Component II is a macromolecule with at least one amino group and Component III is a bulky thiol.
d) Component I wherein R^{1a} is an electron deficient group, Component II has at least four amino groups and Component III is a bulky and small thiol (MW < 200Da).
e) Component I wherein R^{1a} is an electron deficient, an electron donating or a electron neutral group, Component II has at least four amino groups and Component III is a bulky and small thiol (MW < 200 Da).

In a further aspect embodiments of the disclosure relate to an isoindole derivative obtainable by the method as described above in detail.

A yet further aspect embodiments of the disclosure relate to a method for stabilizing an isoindole derivative by producing an isoindole derivative according to the method as described above. In particular, in this method the isoindole derivative is formed in a biological sample, like serum, blood, saliva, urine, milk, juice.

In another aspect, embodiments of the disclosure relate to a method for detecting and/or quantifying a macromolecule comprising a primary amino group, wherein the method comprises:
a) obtaining a stabilized isoindole derivative containing the macromolecule to be detected and/or analyzed according to the method described above; and
b) detecting and/or quantifying said isoindole derivative. Preferably said isoindole derivative is detected and/or quantified by LC methods with UV/fluorescence detection. The skilled person knows methods and materials for detecting the isoindole derivative in particular by LC methods and with UV/fluorescence.

The methods according to the present disclosure allow the control over the stability of isoindole derivatives by using appropriate reaction components, thus enabling not only the detection of different amino containing macromolecules, but also their further functionalization to other useful macromolecular constructs.

The term "stability" as used herein means the tendency of a material to preserve its molecular characteristic over longer time periods (half-life over 24 h) upon change or decomposition due to internal reaction.

In some advantageous embodiments, the highest stability of this type of isoindole derivatives could be achieved through synergetic stabilization factors provided by all three reaction components, i.e. electron withdrawing substituents of 1,2-dialdehyde-π-derivatives, high degree of substitution of amino containing macromolecules inducing most probably a π-stacking stabilization and high degree of substitution of thiol reagents. Less stable products can be obtained when only two of these stabilizing factors are used. Still the products are stable long enough in order to be detected and quantified by LC methods with UV/fluorescence detection. The lower stability of the isoindoles derivatives to nucleophilic attacks with controlled release of thiols could be desirable as delivery systems of selected thiols.

Therefore, in advantageous embodiments the present disclosure pertains to methods for rapid functionalization of macromolecules at room temperature that enables the detection of these macromolecules in complex matrices, as well as their functionalization with different moieties, such as PEG, peptides, and sugars. Combining appropriate reaction components bearing stabilizing moieties, such as electron deficient R^{1a/b} R² with at least one amino group but preferably with more than 4 amino groups and bulky R³ high stability of the isoindole derivatives (with a half-life over 48 h) can be achieved (for R^{1a/b}, R² and R³ see the following reaction scheme 2 and the above explanation of Components I, II, and III).

The derivatization of macromolecules is based on a three component reaction as depicted in Scheme 2 (see alsoFig. 8).

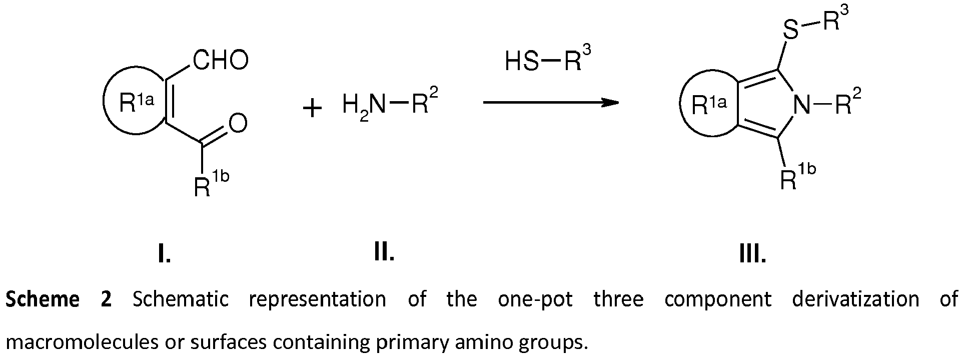

The groups R^{1a} and R^{1b} are as defined above. As to the groups R² and R³, they are derivable from the above given definitions of the Component II and Component III, respectively, because R² and R³ are part of these Components. Thus, also with regard to R² and R³ it is referred to the above description.

The following methods and examples are offered for illustrative purposes only, and are not intended to limit the scope of the present disclosure in any way.

### Methods and Examples

In the following examples, materials and methods of the present disclosure are provided. All publications, patents, and patent applications cited herein are hereby incorporated by reference in their entirety for all purposes.

The highest stability of the isoindole derivatives could be achieved through synergetic stabilization factors provided by all three reaction components, including electron deficient substituents, R^{1a} and/ or R^{1b} of component I, close spatial proximity of amino groups (n>4) for component II and bulky R³ substituent for component III (Scheme 2 above).

Less stable products were obtained when only two of these stabilizing factors were used. Still the products are stable long enough in order to be detected and quantified by LC methods with UV/fluorescence detection. However, a lower stability of the isoindoles derivatives to nucleophilic attacks with controlled release of thiols could be desirable for the preparation of delivery systems able to transport and deliver selected thiols.

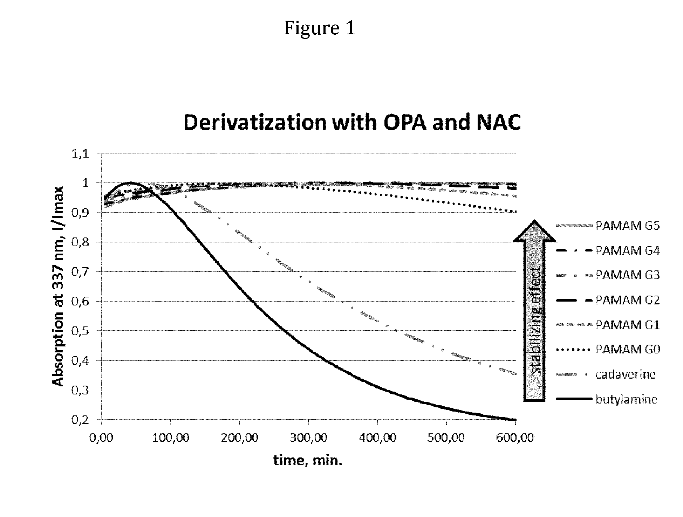

The stability effects of all the factors mentioned above, as well as monitoring of the product formation can be easily probed by measuring the UV absorption (>310nm) of the reaction mixtures at different time intervals since none of the reaction components does absorb at this wavelength range. Thus different reaction partners (component I: OPA, 3,4-diformyl-benzoic acid methyl ester (MOPAC); component II: different generations of PAMAM dendrimers (e.g. generation 0 to generation 5; G0-G5; generation refers to the number of repeated branching cycles that are performed during dendrimer synthesis) and component III: ß-mercaptoethanol (ßMEtOH), N-acetyl-L-cysteine (NAC), poly(ethylene glycol) methyl ether thiol, MW 800) were reacted according to the following general derivatization procedure and the absorption of the reaction mixtures were monitored in time at λₘₐₓ, all intensities being normalized to Iₘₐₓ (Fig. 1-4).

### General procedure for derivatization of macromolecules containing primary amino groups

Thiol reagent (component III) (preferably in buffer solution, pH > 7 and more preferably pH ≥ 8) is added to component II (either in solution or in solid phase) and shaken vigorously for ca. 1 min., then component I (preferably in buffer solution, pH > 7 and more preferably pH ≥ 8) is added and left to react for several minutes. Reaction components are mixed in a ratio of 1/1.1/1.5 with respect to the equivalents of amino groups of component II/ equivalents of component I/ equivalents of component III, respectively whereas larger excess of component I and III are preferable (1/>5/>7.5). The reaction can be carried out over a broad range of temperatures from 0°C up to 80°C preferably at room temperature. Product formation and its stability over time can be easily monitored by UV/fluorescence detection techniques.

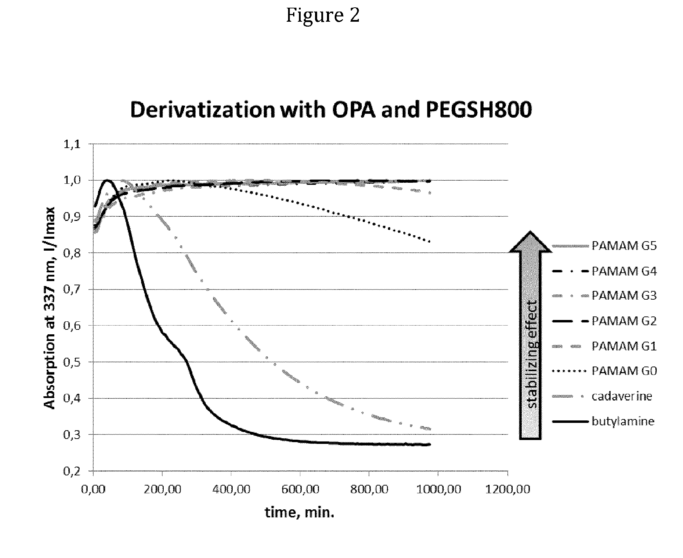

For comparison butylamine and cadaverine as small linear amines were chosen as reference for component II. As can be seen in Fig. 1-4 all reactions reached completion within several minutes, up to about 200 minutes. The UV-absorption versus time plots revealed distinct differences in stability of the reaction products, evidencing the stabilization effects induced by the three components. Thus the higher the density of the amino groups of component II, the more stable is the corresponding isoindole product (Fig.1, and 2).

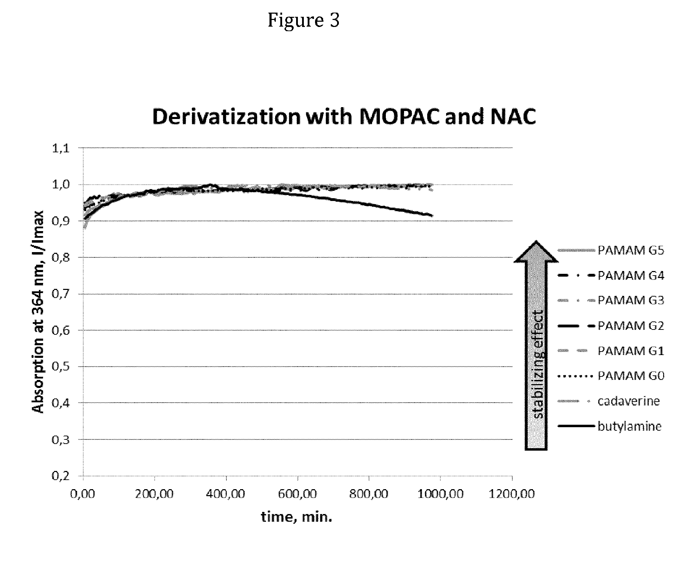

The higher the electron withdrawing effects of R^{1a} and R^{1b} the more stable is the corresponding isoindole product (i.e. OPA vs MOPAC, Fig.1 and 3). The bulkier R³ of component III the more stable is the corresponding isoindole product.

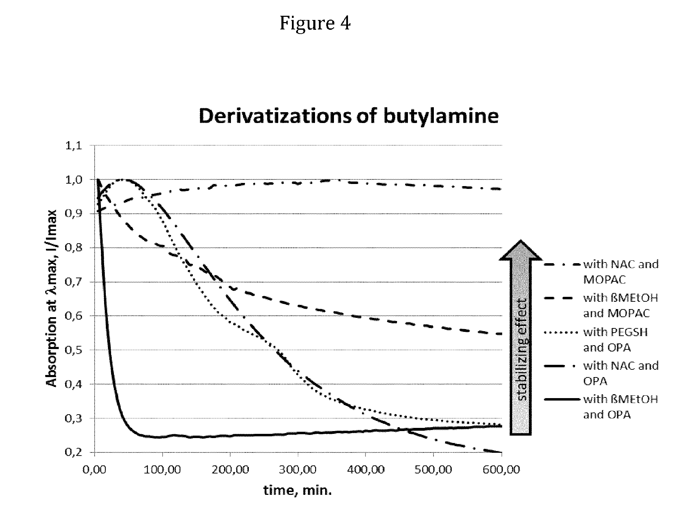

The last effect could be easier followed up by comparing the stability of isoindoles products of butylamine derivatized with two thiol reagents such as, β-MEtOH and NAC (Fig. 4).

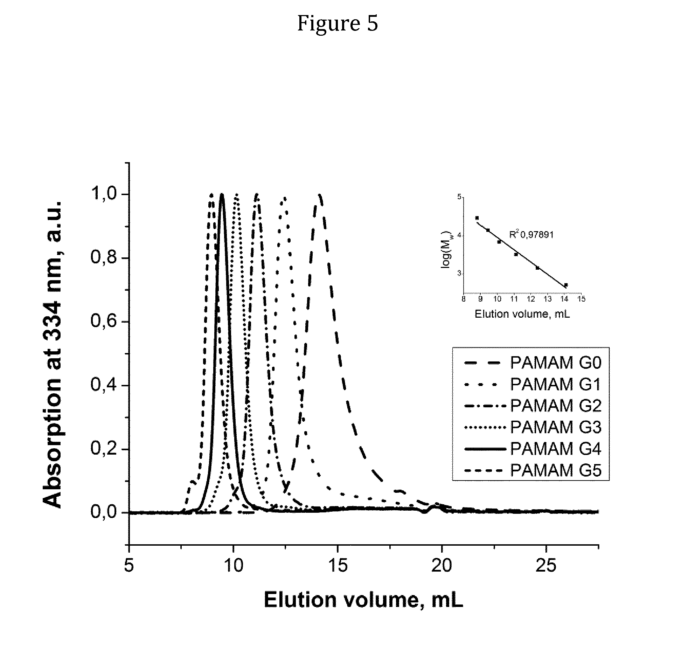

The derivatization method for functionalization, detection and quantitation of macromolecules has been successfully applied to PAMAM dendrimers of different generations, as model macromolecules for component II with various numbers of primary amino groups. The reaction mixtures were monitored by Size Exclusion - Fast Protein Liquid Chromatography (SE-FPLC) with UV/fluorescence detection showing distinct retention times for different generations of dendrimers, according to their molecular weight after derivatization with Component I (OPA) and III (PEG-SH₈₀₀) in aqueous solution (Fig. 5).

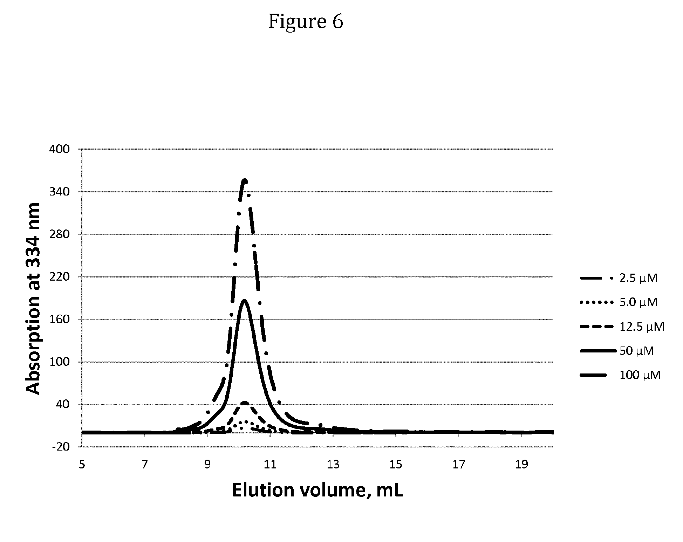

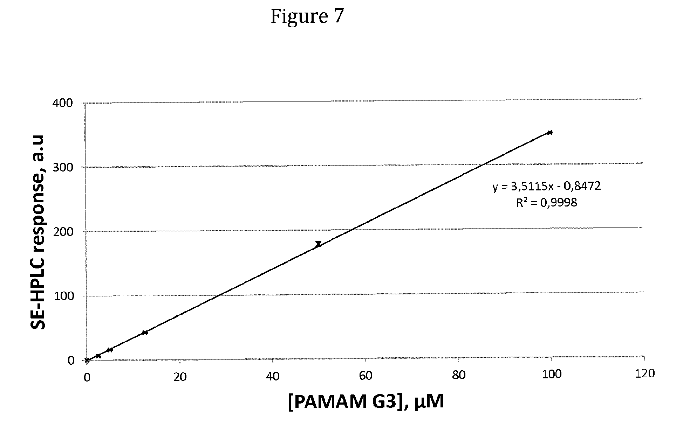

Moreover the method allows the quantification of the macromolecules by using different Liquid Chromatography (LC) techniques with UV/fluorescence detection. Thus measuring the SE-FPLC chromatograms of the reaction mixtures of different concentrations of component II (Fig. 6) and plotting the signal area (or height) of the peaks versus concentration (Fig. 7) a good linearity for a broad concentration range of the derivatized macromolecule can be obtained.

In conclusion, the invention provides a method for rapid functionalization of macromolecules at room temperature with reagents that enables the detection of these macromolecules in complex matrices, as well as their functionalization with different moieties, such us PEG, peptides, and sugars. Combining appropriate reaction components bearing stabilizing moieties, such as electron deficient R¹, R² with at least one amino group but preferably with more than 4 amino groups and bulky R³ high stability of the isoindole derivatives (over 48 h) can be achieved.

### REFERENCES

The contents of all cited references, including literature references, issued patents, and published patent applications, as cited throughout this application are hereby expressly incorporated by reference.
Q. Zhang, Y. Sha, J.-H. Wang Molecules, 2010, 15, 2969-2971.
B.-B. Wang, X. Zhang, X.-R. Jia, Z.-C. Li, Y. Ji, L. Yang, Y. Wie J. Am. Chem. Soc. 2004, 126, 15180-15194.
M. Roth, Anal. Chem. 1971, 43, 880-882.
P. Zuman, Chem. Rev. 2004, 104, 3217-3238.
D. P. Manica, J. A. Lapos, A. D. Jones, A. G. Ewing Anal. Biochem. 2003, 322, 68-78.
W.A. Jacobs, M.W. Leburg, E.J. Madaj, Anal. Biochem. 1986, 156, 334-340.
R. Hanczkó, A. Jámbor, A. Perl, I. Molnár-Perl J. Chromatogr. A 2007, 1163, 25-42.
T. I. F. Cremers, J. L. A. Anna De, W. S. Faber, Method for the determination of an analyte comprising a primary amino group, and kit for labeling said analyte, WO2008094043 A2, 2008**,** PCT/NL2008/050155.
O. Richard, R. Gavin, S. Richard, Method for analysing amino acids, peptides and proteins using mass spectroscopy of fixed charge-modified derivatives, WO 2004046731 A3, 2005**,** PCT/US2003/036739

## Claims

1. A method for producing an isoindole derivative, wherein a Component I having the formula 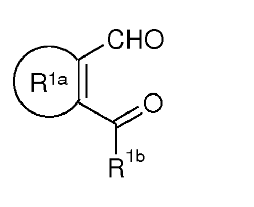 in which
R^{1b} is H, C1 to C4 alkyl group or an electron deficient aromatic group, and 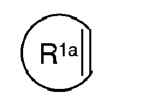
is an electron deficient π-system, an electron neutral π-system or an electron rich π-system,
is reacted in an one-pot-reaction with, as Component II, a macromolecule having at least one primary amino group or an amino functionalized surface and, as Component III, a thiol reagent or a thiol functionalize surface.

2. The method according to claim 1, wherein the electron deficient π-system is selected from the group consisting of pyridine, pyrimidine, benzoic acid, benzonitrile, (1,1',1"-trifluoromethyl)benzene, benzeneboronic acid, nitrobenzene, chlorobenzene, quinoline, isoquinoline, naphthyridine, the electron neutral π-system is benzene or naphthalene, and the electron rich π-system is selected from the group consisting of toluene, thiophene, phenol, furan, pyrrole, N,N-dimethylaniline.

3. The method according to claim 1 or 2, wherein Component I is selected from the group consisting of 2,3-pyridinedicarboxyaldehyde, 3,4-diformyl-benzoic acid, 2-acetylthiophene-3-carboxyaldehyd or 4-(2-formylquinoline-3-carbonyl)benzoic acid.

4. The method according to any of the preceding claims, wherein the macromolecule as Component II has at least four amino groups.

5. The method according to any of the preceding claims, wherein the component II is selected from the group consisting of polylysine, poly(amidoamine) dendrimers, polypropylene imine dendrimers, poly(melamine) dendrimers, peptides, proteins, and sugars.

6. The method according to any of the preceding claims, wherein the thiol as Component III contains at least one thiol group and no primary amino group.

7. The method according to any of the preceding claims, wherein the reaction comprises the steps of:
a), adding a solution of component III to component II that can be either in solution or in solid phase (in the case of amino functionalized surfaces), or in case that component III is in solid phase (thiol functionalized surface) a solution of component II is added to component III,
b) adding Component I to the mixture obtained in step a), and
c) incubating the reaction mixture of Component I, II and III obtained after step b) in order to obtain the isoindole derivative.

8. The method according to any of the preceding claims, wherein the Components are mixed in a ratio of 1 : 1.1 : 1.5 with respect to the equivalents of amino groups of Component II : equivalents of Component I : equivalents of Component III.

9. The method according to any of the preceding claims, wherein the Components are mixed with a larger excess of Component I and III, preferably in a ration 1 : >5 : >7.5 with respect to the equivalents of amino groups of Component II : equivalents of Component I : equivalents of Component III.

10. The method according to any of the preceding claims, wherein the reaction of Component I, II and III is carried out in an aqueous solution having a pH > 7 and preferably having a pH ≥ 8.

11. The method according to any of the preceding claims, wherein the method is carried out at a temperature in a range from 0 °C to 80 °C, preferably room temperature.

12. The method according to any of the preceding claims, wherein
a) Component I is 3,4-diformyl-benzoic acid methyl ester or o-phthaldialdehyde,
b) Component II is a protein or a dendrimer like a poly(amidoamine) dendrimer
c) Component III is β-mercaptoethanol, N-acetyl-L-cysteine, poly(ethylene glycol) methyl ether thiol having a molecular weight of about 800.

13. An isoindole derivative obtainable by a method according to any of the preceding claims.

14. A method for stabilizing an isoindole derivative by producing an isoindole derivative according to the method as defined in any of claims 1 to 12.

15. The method of claim 14, wherein the isoindole derivative is formed in a biological sample.

16. A method for detecting and/or quantifying a macromolecule comprising a primary amino group, wherein the method comprises:
a) obtaining a stabilized isoindole derivative containing the macromolecule to be detected and/or analyzed according to the method as defined in any of the claims 1 to 12; and
b) detecting and/or quantifying said isoindole derivative.

17. The method of claim 16, wherein said isoindole derivative is detected and/or quantified by LC methods with UV/fluorescence detection.
